(19) Europäisches Patentamt
European Patent Office
Office européen des brevets

(11) Veröffentlichungsnummer: **0 243 607**
**B1**

(12) # EUROPÄISCHE PATENTSCHRIFT

(45) Veröffentlichungstag der Patentschrift: **09.05.90**

(51) Int. Cl.[5]: **C25B 3/02,** C07C 269/00

(21) Anmeldenummer: **87102688.6**

(22) Anmeldetag: **25.02.87**

(54) **Verfahren zur Herstellung von Biscarbamaten.**

(30) Priorität: **28.02.86 DE 3606478**

(43) Veröffentlichungstag der Anmeldung:
**04.11.87 Patentblatt 87/45**

(45) Bekanntmachung des Hinweises auf die Patenterteilung:
**09.05.90 Patentblatt 90/19**

(84) Benannte Vertragsstaaten:
**BE CH DE FR GB IT LI NL SE**

(56) Entgegenhaltungen:
**EP-A- 0 067 463**
**DE-A- 1 965 101**

**JOURNAL OF THE CHEMICAL SOCIETY, Chemical Communications, Nr. 16, 18. August 1976, Seiten 640-642; L.C. HODGKINSON et al.:"Formation of complexes between macrocyclic diamines and primary ammonium thiocyanates: Dependence of binding upon host structure"tion" page 330, column 2, abstract no. 71 896b4, 17(9), 11-15 000**

(73) Patentinhaber: **BASF Aktiengesellschaft, Carl-Bosch-Strasse 38, D-6700 Ludwigshafen(DE)**

(72) Erfinder: **Degner, Dieter, Dr., Kurpfalzstrasse 8, D-6701 Dannstadt-Schauernheim(DE)**
Erfinder: **Hannebaum, Heinz, Ostring 56, D-6700 Ludwigshafen(DE)**

**EP 0 243 607 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die vorliegende Erfindung betrifft ein neues Verfahren zur Herstellung von Biscarbamaten.

Bekanntlich stellt man Carbamidsäureester durch Umsetzung von Alkoholen mit Phosgen zu Chlorameisensäureestern und anschließende Aminolyse her. Der Umgang mit den hochtoxischen und korrosiven Vor- und Zwischenprodukten erfordert einen erheblichen technischen Aufwand. Weiterhin fallen bei diesen Verfahren Salzsäure oder halogenhaltige Abfallsalze an, deren Abtrennung häufig technisch sehr aufwendig ist (Ullmann, Enzyklopädie der techn. Chemie, 4. Auflage, Bd. 9, S. 118-119).

Nach einer phosgenfreien Herstellungsmethode wird Harnstoff mit Alkanolen umgesetzt. Nachteilig sind hierbei die hohen Reaktionstemperaturen und langen Reaktionszeiten sowie der technisch umständliche Umgang mit Feststoffen (Houben-Weyl, Methoden der organischen Chemie, 1952, Bd. 8, S. 14O).

Der Erfindung lag nun die Aufgabe zugrunde, ein Verfahren zu finden, das es ermöglicht, Biscarbamidsäureester technisch einfacher und auf besonders umweltfreundliche Art herzustellen.

Nach dem erfindungsgemäßen Verfahren, durch das diese Aufgabe gelöst wurde, stellt man Biscarbamate der allgemeinen Formel

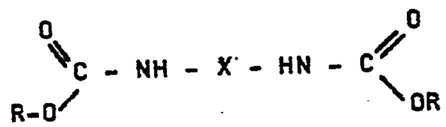

I,

in der R für einen Alkylrest mit 1 bis 8 C-Atomen steht und X einen Kohlenwasserstoffrest mit 2 bis 6O C-Atomen bedeutet, in dem ein oder mehrere C-Atome durch Heteroatome ersetzt sein können und der Halogenatome, Ester-, Carbonyl- oder Nitrilgruppen enthalten kann, dadurch her, daß man Formamide der allgemeinen Formel

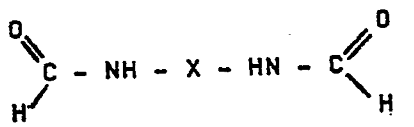

II,

in der X die obengenannte Bedeutung hat, in Gegenwart eines Alkanols der Formel ROH, in der R die obengenannte Bedeutung hat, und eines ionogenen Halogenids elektrochemisch oxidiert.

Die als Ausgangsstoffe für die Herstellung der Biscarbamate der Formel I genannten Formamide der Formel II enthalten als zweiwertigen Rest X einen geradkettigen, verzweigten oder ringförmigen Kohlenwasserstoffrest mit 2 bis 6O, vorzugsweise 3 bis 3O, insbesondere 3 bis 12 C-Atomen, wie einen Alkylrest, einen Cycloalkylrest oder einen Alkylarylrest. In diesen Kohlenwasserstoffresten können ein oder mehrere C-Atome durch Heteroatome, wie N, O oder S ersetzt sein. Die genannten Kohlenwasserstoffreste können auch Halogenatome, Ester-, Carbonyl- oder Nitrilgruppen tragen.

Beispielsweise seien die folgenden Formamide genannt:

$OHC-NH-(CH_2)_n-NHCHO$ IV

mit n = 3 bis 30

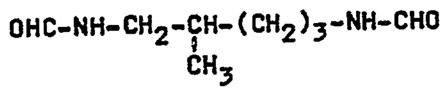

V

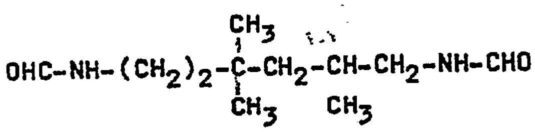

VI

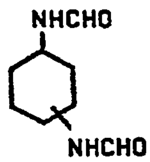

VII

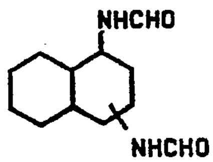

VIII

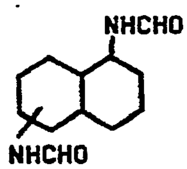

IX

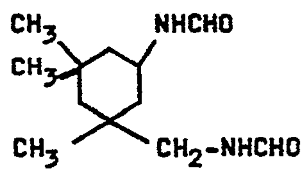

X

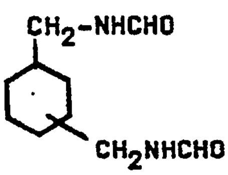

XI

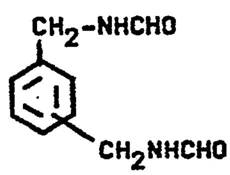

XII

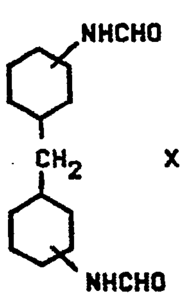

XIII

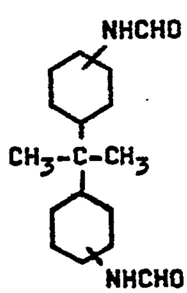

XIV

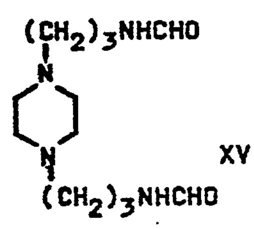

XV

$$OHCHN-(CH_2)_n-\underset{}{\overset{CHO}{N}}-(CH_2)_m-NHCHO \qquad XVI$$

mit n und m = 2 bis 30

$$OHCHN-(CH_2)_n-O\left[(CH_2)_q-O\right]_p(CH_2)_m-NHCHO \qquad XVII$$

mit n, m, q = 2 bis 30 und p = 1 bis 4

In den Alkoholen der Formel ROH steht R für einen Alkylrest mit 1 bis 8, vorzugsweise 1 bis 5, insbesondere 1 bis 4 Kohlenstoffatomen. Als Alkanole seien beispielsweise n- oder iso-Propanol, n-Butanol, insbesondere aber Methanol und Ethanol genannt.

Als ionogene Halogenide kommen Salze, wie die Alkali-, Erdalkali- oder Ammoniumsalze der Iodwasserstoff-, Bromwasserstoff- und Chlorwasserstoffsäure in Betracht. Besonders bevorzugt sind Salze der Bromwasserstoffsäure, wie Alkali- und Erdalkalibromide sowie quaternäre Ammonium-, insbesondere Tetraalkylammoniumbromide. Das Kation spielt keine erfindungswesentliche Rolle, es können daher auch andere ionogene Metallhalogenide verwendet werden. Zweckmäßigerweise wird man billige Halogenide wählen. Beispielsweise seien Natrium-, Kalium-, Calcium- und Ammoniumbromid sowie Di-, Tri- und Tetramethyl- oder Tetraethylammoniumbromid genannt.

Das erfindungsgemäße Verfahren erfordert keine besondere Elektrolysezelle. Vorteilhaft kann man es in einer ungeteilten Durchflußzelle durchführen. Als Anoden können alle an sich üblichen Anodenmaterialien verwendet werden, die unter den Elektrolysebedingungen stabil sind, wie Edelmetalle, z.B. Gold oder Platin. Bevorzugtes Anodenmaterial ist Graphit. Das Kathodenmaterial besteht z.B. aus Metallen, wie Blei, Eisen, Stahl, Nickel oder Edelmetallen wie Platin. Bevorzugtes Kathodenmaterial ist ebenfalls Graphit.

Die Zusammensetzung des Elektrolyten kann in weiten Grenzen gewählt werden. Beispielsweise enthält der Elektrolyt die Ausgangsstoffe in folgenden Gewichtsverhältnissen:
1 bis 50 Gew.% Formamid der Formel II
40 bis 90 Gew.% Alkanol
0,1 bis 10 Gew.% Halogenid.

Dem Elektrolyten kann, so gewünscht, ein Lösungsmittel, etwa zur Verbesserung der Löslichkeit des Formamids oder des Halogenids, zugesetzt werden. Geeignete Lösungsmittel sind Nitrile, wie Acetonitril, Carbonate, wie Dimethylcarbonat, Ether, wie Tetrahydrofuran und Dialkylformamide wie Dimethylformamid. Die Stromdichte ist kein begrenzter Faktor für das erfindungsgemäße Verfahren, sie beträgt z.B. 1 bis 25 A/dm², vorzugsweise wird mit einer Stromdichte von 3 bis 12 A/dm² elektrolysiert. Man elektrolysiert z.B. bei Temperaturen bis 120°C. Bei druckloser Fahrweise der Elektrolyse wählt man einen Temperaturbereich, dessen oberer Wert zweckmäßigerweise zumindest 5 bis 10°C unter dem Siedepunkt des Elektrolyten liegt. Bei Verwendung von Methanol oder Ethanol wird vorzugsweise bei Temperaturen von 20 bis 50°C elektrolysiert.

Die Aufarbeitung der Elektrolyseausträge kann man nach an sich bekannten Methoden vornehmen. Zweckmäßigerweise wird der Elektrolyseaustrag zunächst destillativ aufgearbeitet. Überschüssiges Alkanol und evtl. eingesetztes Kolösungsmittel werden abdestilliert. Die Halogenide werden in bekannter Weise, z.B. durch Filtration oder Extraktion abgetrennt. Die Biscarbamate fallen danach häufig in sehr reiner Form an, sie können bei Bedarf, z.B. durch Umfällen oder Umkristallisation weiter gereinigt werden. Alkanol, evtl. noch unumgesetztes Formamid, Kolösungsmittel sowie Halogenide können zur Elektrolyse zurückgeführt werden. Das erfindungsgemäße Verfahren kann sowohl diskontinuierlich als auch kontinuierlich durchgeführt werden.

Nach dem erfindungsgemäßen Verfahren erhält man die Biscarbamate auf besonders vorteilhafte Weise. Das neue Verfahren bietet überraschenderweise die Möglichkeit, die Formamide weitgehend umzusetzen, ohne daß es zu Ausbeuteverschlechterungen kommt. Auch die Stromausbeuten sind bei dem erfindungsgemäßen Verfahren ungewöhnlich hoch. So ist das Formamid bei der Elektrolyse mit 4 bis 5 F/Mol Formamid meist bereits vollständig umgesetzt. Diese günstigen Resultate waren nicht zu erwarten. Es ist nämlich seit langem bekannt, daß die elektrochemische Umsetzung von Formamiden in Alkoholen in Gegenwart von Leitsalzen wie Tetraalkylammoniumtetrafluoroborat zu Alkoxiformamiden führt (s. Tetrahedron 32 (1976), S. 2185-2206), und daß Biscarbamate unter Elektrolysebedingungen bei Verwendung von Leitsalzen wie Tetraethylammonium-p-toluolsulfonat elektrochemisch weiteroxidiert werden (s. J. Org. Chem. (1983), 48, S. 3338-3339).

Die Biscarbamate sind vielfältig einsetzbare Zwischenprodukte für die Synthese von Isocyanaten.

Beispiel 1 Elektrosynthese von $CH_3OOCHN-(CH_2)_6NHCOOCH_3$

| | | |
|---|---|---|
| Apparatur: | ungeteilte Zelle mit 11 Elektroden | |
| Anode: | Graphit | |
| Elektrolyt: | 300 g Formamid der Formel IV mit n = 6 | (8,5 Gew.-%) |
| | 36 g NaBr | (1,0 Gew.-%) |
| | 3204 g $CH_3OH$ | (90,5 Gew.-%) |
| Kathode: | Graphit | |
| Stromdichte: | 3,3 $A/dm^2$ | |
| Temperatur: | 25°C | |
| Elektrolyse mit | 5,3 F/Mol des Formamids | |
| Durchfluß durch Zelle: | 200 l/h | |

Aufarbeitung:
Nach Beendigung der Elektrolyse wird Methanol bei Normaldruck abdestilliert und der Rückstand in Methylethylketon heiß gelöst. Das Natriumbromid (36 g) wird heiß abfiltriert. Das Reaktionsprodukt wird aus dem Filtrat auskristallisiert. Dabei erhält man 385 g $CH_3OOC-NH-(CH_2)_6-NHCOOCH_3$ (Fp. 112°C, nach $^1$HNMR-Spektrum rein, Analyse für $C_{10}H_{20}N_2O_4$ (%); ber.: C:51.7, H:8.6, O:27.6, N:12.1; gef.: C:51.7, H:8.5, O:27.6, N:12.O). Dies entspricht einer Ausbeute von 95.1 %.

Beispiel 2Elektrosynthese von $CH_3OCONH-(CH_2)_4NHCOOCH_3$

| | | |
|---|---|---|
| Apparatur: | ungeteilte Zelle mit 6 Elektroden | |
| Anode: | Graphit | |
| Elektrolyt: | 300 g Formamid der Formel IV mit n = 4 | (10,0 Gew.-%) |
| | 30 g NaBr | (1,0 Gew.-%) |
| | 2760 g $CH_3OH$ | (89,0 Gew.-%) |
| Kathode: | Graphit | |
| Stromdichte: | 3,3 $A/dm^2$ | |
| Temperatur: | 25°C | |
| Elektrolyse mit | 4,75 F/Mol des Formamids | |
| Durchfluß durch Zelle: | 200 l/h | |

Aufarbeitung:
Man arbeitet analog Beispiel 1 auf erhält neben 27g Natriumbromid 356g $CH_3OCONH-(CH_2)_4-NH-COOCH_3$ (Fp. 126 bis 128°C, nach $^1$HNMR-Spektrum rein). Dies entspricht einer Ausbeute von 83,8%.

Beispiel 3 Elektrosynthese von $CH_3OCONH-(CH_2)_5-NHCOOCH_3$

| | | |
|---|---|---|
| Apparatur: | ungeteilte Zelle mit 6 Graphitelektroden | |
| Anode: | Graphit | |
| Elektrolyt: | 140 g Formamid der Formel IV mit n = 5 | (4,7 Gew.-%) |
| | 30 g NaBr | (1,0 Gew.-%) |
| | 2830 g $CH_3OH$ | (94,3 Gew.-%) |
| Kathode: | Graphit | |
| Stromdichte: | 3,3 $A/dm^2$ | |
| Temperatur: | 25°C | |
| Elektrolyse mit | 4,4 F/Mol des Formamids | |
| Durchfluß durch Zelle: | 200 l/h | |

Aufarbeitung:

Man arbeitet analog Beispiel 1 auf und erhält neben 30 g Natriumbromid 175 g $CH_3OCONH-(CH_2)_5-NHCOOCH_3$ (Fp. 104 bis 112°C; nach $^1$HNMR-Spektrum rein, Analyse ber. für $C_9H_{18}N_2O_4$ (%); ber.: C:49.5, H:8.3, O:29.4, N:12.8; gef.: C:49.5, H:8.1, O:29.4, N:12.8). Dies entspricht einer Ausbeute von 90.6 %.

Beispiel 4 Elektrosynthese von

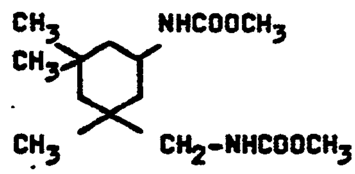

| | | |
|---|---|---|
| Apparatur: | ungeteilte Zelle mit 11 Elektroden | |
| Anode: | Graphit | |
| Elektrolyt: | 279.2 g Formamid der Formel X | (8,8 Gew.-%) |
| | 32 g NaBr | (1,0 Gew.-%) |
| | 2848 g $CH_3OH$ | (90,2 Gew.-%) |
| Kathode: | Graphit | |
| Stromdichte: | 3,3 A/dm$^2$ | |
| Temperatur: | 20–22°C | |
| Elektrolyse mit | 4,5 F/Mol des Formamids | |
| Durchfluß durch Zelle: | 200 l/h | |

Aufarbeitung:

Nach Beendigung der Elektrolyse wird Methanol bei Normaldruck abdestilliert. Der feste Rückstand wird in Methylethylketon aufgenommen und der zurückbleibende Feststoff (32 g, NaBr) abfiltriert. Aus dem Filtrat wird Methylethylketon abdestilliert. Der Rückstand wird aus wenig Methylethylketon umkristallisiert. Hierbei erhält man 282 g des Biscarbamates (Fp. 103 bis 117°C; nach $^1$HNMR- und $^{13}$C-NMR-Spektren rein, cis-trans-Gemisch). Dies entspricht einer Ausbeute von 79,8 %.

Beispiel 5 Elektrosynthese von

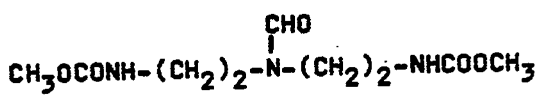

| | | |
|---|---|---|
| Apparatur: | ungeteilte Zelle mit 11 Elektroden | |
| Anode: | Graphit | |
| Elektrolyt: | 330 g Formamid der Formel XVI mit n = m = 2 | (10 Gew.-%) |
| | 33 g NaBr | (1 Gew.-%) |
| | 2937 g $CH_3OH$ | (89 Gew.-%) |
| Kathode: | Graphit | |
| Stromdichte: | 3,3 A/dm$^2$ | |
| Temperatur: | 25°C | |
| Elektrolyse mit | 4,5 F/Mol des Formamids | |
| Durchfluß durch Zelle: | 200 l/h | |

Aufarbeitung:
Nach Beendigung der Elektrolyse wird Methanol bei Raumtemperatur abdestilliert. Der Rückstand wird in Methylenchlorid aufgenommen und Natriumbromid (28 g) abfiltriert. Aus dem Filtrat wird Methylenchlorid abdestilliert. Der verbleibende Rückstand (4O8 g) wird dann aus Methylethylketon umkristallisiert. Hierbei erhält man 248,2 g des Biscarbamates (Fp. 9O bis 92°C; nach $^1$HNMR- und $^{13}$-Spekten rein, Analyse für $C_9H_{17}N_3O_5$ ber. (%): C:43.7, H:6.9, O:32.4, N:17.O; gef.: C:43.6, H:6.8, O:32.1, N:17.1). Aus der Mutterlauge können weitere 128,5 g des Biscarbamates isoliert werden. Dies entspricht einer Gesamtausbeute von 86,4 %.

Beispiel 6 Elektrosynthese von $CH_3OOCHN-(CH_2)_3-O-(CH_2)_4-O-(CH_2)_3-NHCOOCH_3$

| | | |
|---|---|---|
| Apparatur: | ungeteilte Zelle mit 11 Elektroden | |
| Anode: | Graphit | |
| Elektrolyt: | 300 g Formamid der Formel XVII mit n = m = 3, q = 4, p = 1 | (10 Gew.-%) |
| | 33 g NaBr | (1 Gew.-%) |
| | 2937 g $CH_3OH$ | (89 Gew.-%) |
| Kathode: | Graphit | |
| Stromdichte: | 3,3 $A/dm^2$ | |
| Temperatur: | 25°C | |
| Elektrolyse mit | 4,75 F/Mol des Formamids | |
| Durchfluß durch Zelle: | 200 l/h | |

Aufarbeitung:
Nach Beendigung der Elektrolyse wird Methanol bei Normaldruck abdestilliert. Der Rückstand wird in Methylethylketon aufgenommen, dann wird Natriumbromid (32 g) abfiltriert und Methylethylketon abdestilliert. Hierbei erhält man 357 g $CH_3OCONH-(CH_2)_3-O-(CH_2)_4-O-(CH_2)_3-NHCOOCH_3$ (Fp. 45 bis 46°C; nach $^1$HNMR- und $^{13}$CNMR-Spektren rein, Analyse ber. für $C_{14}H_{28}N_2O_6$ (%): C:52.5, H:8.8, O:3O.O, N:8.8; gef.: C:52.7, H:8.7, O:29.6, N:8.8). Dies entspricht einer Ausbeute von 87.9 %.

Beispiel 7 Elektrosynthese von $CH_3OCONH-(CH_2)_3-O-[(CH_2)_2-O]_2-(CH_2)_3-NHCOOCH_3$

| | | |
|---|---|---|
| Apparatur: | ungeteilte Zelle mit 6 Elektroden | |
| Anode: | Graphit | |
| Elektrolyt: | 260 g Formamid der Formel XVII mit n = m = 3, q = 2, p = 2 | (10 Gew.-%) |
| | 26 g NaBr | (1 Gew.-%) |
| | 2314 g $CH_3OH$ | (89 Gew.-%) |
| Kathode: | Graphit | |
| Stromdichte: | 3,3 $A/dm^2$ | |
| Temperatur: | 20 bis 22°C | |
| Elektrolyse mit | 4,75 F/Mol des Formamids | |
| Durchfluß durch Zelle: | 200 l/h | |

Aufarbeitung:
Nach Beendigung der Elektrolyse wird Methanol bei Normaldruck abdestilliert. Der Rückstand wird in Toluol aufgenommen, dann wird Natriumbromid (22 g) abfiltriert und Toluol wieder abdestilliert. Hierbei erhält man 275 g $CH_3OOCNH(CH_2)_3-O-(CH_2)_2-O-(CH_2)_2-O-(CH_2)_3NHCOOCH_3$ (viskoses Öl, nach $^1$HNMR- und $^{13}$CNMR-Spektren rein, Analyse für $C_{14}H_{28}N_2O_7$ berechnet (%): C:5O.O, H:8.3, O:33.4, N:8.3; gef.: C:5O.2, H:8.2, O:33.3, N:8.5). Dies entspricht einer Ausbeute von 86.9 %.

Beispiel 8 Elektrosynthese von

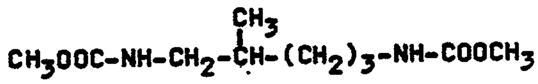

| | | |
|---|---|---|
| Apparatur: | ungeteilte Zelle mit 6 Elektroden | |
| Anode: | Graphit | |
| Elektrolyt: | 260 g Formamid der Formel V | (10 Gew.-%) |
| | 26 g NaBr | (1 Gew.-%) |
| | 2314 g $CH_3OH$ | (89 Gew.-%) |
| Kathode: | Graphit | |
| Stromdichte: | 3,3 $A/dm^2$ | |
| Temperatur: | 27°C | |
| Elektrolyse mit | 4,5 F/Mol des Formamids | |
| Durchfluß durch Zelle: | 200 l/h | |

Aufarbeitung:
Nach Beendigung der Elektrolyse wird Methanol bei Normaldruck abdestilliert. Der Rückstand wird mit Methyl-tert.-butylether aufgenommen und Natriumbromid (24 g) abfiltriert. Aus dem Filtrat wird Methyl-tert.-butylether abdestilliert. Hierbei erhält man 29O g $CH_3OOC$-NH-$CH_2$-CH($CH_3$)-($CH_2$)$_3$-NH-$COOCH_3$ (gelbe Flüssigkeit, nach $^1$HNMR- und $^{13}$CNMR-Spektren rein, Analyse ber. für $C_{10}H_{20}N_4O_2$ (%): C:51.7, H:8.6, O:27.6, N:12.1; gef.: C:51.7, H:8.7, O:27.7, N:12.5). Dies entspricht einer Ausbeute von 82,7 %.

Beispiel 9 Elektrosynthese von

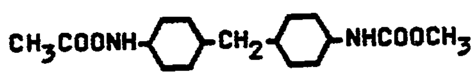

| | | |
|---|---|---|
| Apparatur: | ungeteilte Zelle mit 6 Elektroden | |
| Anode: | Graphit | |
| Elektrolyt: | 86 g 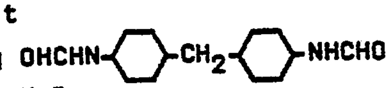 | (3,5 Gew.%) |
| | 26 g NaBr | (1,1 Gew.%) |
| | 2314 g $CH_3OH$ | (95,4 Gew.%) |
| Kathode: | Graphit | |
| Stromdichte: | 3,3 $A/dm^2$ | |
| Temperatur: | 43 bis 50°C | |
| Elektrolyse mit | 4,5 F/Mol des Formamids | |
| Durchfluß durch Zelle: | 200 l/h | |

Aufarbeitung:
Nach Beendigung der Elektrolyse wird der Elektrolyt auf 1O°C abgekühlt. Dabei fallen 38.4 g

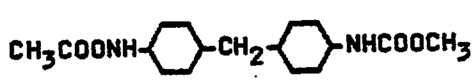

(Fp. 195 bis 2OO°C, nach$^1$HNMR- und $^{13}$C-NMR-Spektren reines cis-trans-Gemisch) aus. Danach wird Methanol bei Normaldruck abdestilliert, der Rückstand in $CH_2Cl_2$ aufgenommen, NaBr (24,5 g) abfil-

triert und $CH_2Cl_2$ abdestilliert. Hierbei erhält man weitere 51 g des Biscarbamates (Fp. 168 bis 173°C). Dies entspricht einer Gesamtausbeute von 84,8 %.

Beispiel 10 Elektrosynthese von

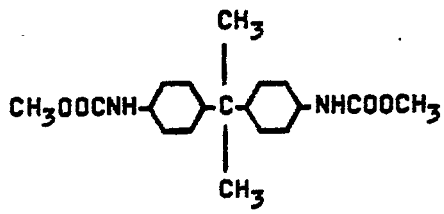

| | | |
|---|---|---|
| Apparatur: | ungeteilte Zelle mit 6 Elektroden | |
| Anode: | Graphit | |
| Elektrolyt: | 260 g 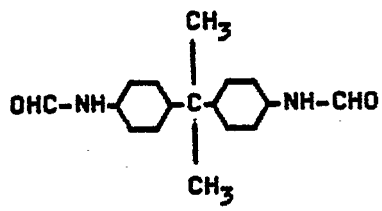 | (10 Gew.%) |
| | 26 g NaBr | ( 1 Gew.%) |
| | 2314 g $CH_3OH$ | (89 Gew.%) |
| Kathode: | Graphit | |
| Stromdichte: | 3,3 A/dm$^2$ | |
| Temperatur: | 27°C | |
| Elektrolyse mit | 4,0 F/Mol des Formamids | |
| Durchfluß durch Zelle: | 200 l/h | |

Aufarbeitung:
Nach Beendigung der Elektrolyse wird Methanol bei Normaldruck abdestilliert. Der Rückstand wird in Diethylketon gelöst und mit Wasser gewaschen. Die organische Phase wird mit Natriumsulfat getrocknet, dann wird Diethylketon abdestilliert. Hierbei erhält man 2O6,7 g des Biscarbamates der oben angegebenen Formel. (Fp. 137 bis 145°C, nach $^1$HMR- und $^{13}$CNMR-Spektren reines cis-trans-Gemisch, Analyse ber. für $C_{19}H_{34}O_4N_2$ (%): C:64.4, H:9.6, O:18.1, N:7.9; gef.: C:64.3, H:9,8, O:18.O, N:7.9). Dies entspricht einer Ausbeute von 66 %.

Beispiel 11 Elektrosynthese von

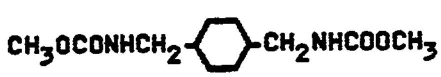

| | |
|---|---|
| Apparatur: | ungeteilte Zelle mit 6 Elektroden |
| Anode: | Graphit |
| Elektrolyt: | 31.6 g 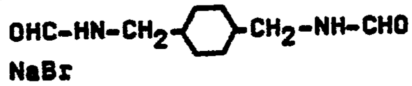 (1,3 Gew.%) |
| | 26 g NaBr (1,1 Gew.%) |
| | 2314 g $CH_3OH$ (97,6 Gew.%) |
| Kathode: | Graphit |
| Stromdichte: | 3,3 A/$dm^2$ |
| Temperatur: | 25 bis 27°C |
| Elektrolyse mit | 4,5 F/Mol des Formamids |
| Durchfluß durch Zelle: | 200 l/h |

Aufarbeitung:
Nach Beendigung der Elektrolyse wird Methanol bei Normaldruck abdestilliert. Der Rückstand wird mit Methylenchlorid aufgenommen. Natriumbromid (25 g) wird abfiltriert und Methylenchlorid abdestilliert. Hierbei erhält man 29.9 g des Biscarbamates der oben angegebenen Formel (Schmelzbereich 144 bis 167°C, nach $^1$HNMR- und $^{13}$CNMR-Spektren reines cis-trans-Gemisch, Analyse ber. für $C_{12}H_{22}N_2O_4$ (%): C:55.8, H:8.6, O:24.8, N:10.8; gef.: C:55.7, H:8.5, O:24.8, N:10.9). Dies entspricht einer Ausbeute von 72,6 %.

Beispiel 12 Elektrosynthese von

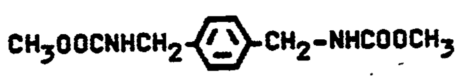

| | |
|---|---|
| Apparatur: | ungeteilte Zelle mit 6 Elektroden |
| Anode: | Graphit |
| Elektrolyt: | 80 g 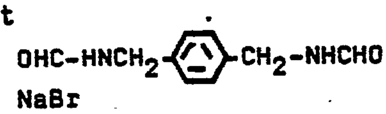 ( 3,0 Gew.%) |
| | 26 g NaBr ( 1,0 Gew.%) |
| | 1357 g $(CH_3)_2NCHO$ (51,8 Gew.%) |
| | 1157 g $CH_3OH$ (44,2 Gew.%) |
| Kathode: | Graphit |
| Stromdichte: | 3,3 A/$dm^2$ |
| Temperatur: | 27°C |
| Elektrolyse mit | 6,6 F/Mol des Formamids |
| Durchfluß durch Zelle: | 200 l/h |

Aufarbeitung:
Nach Beendigung der Elektrolyse wird zunächst Methanol bei Normaldruck, danach Dimethylformamid bei 15 mbar Kopfdruck bis 110°C (Sumpftemperaturen) abdestilliert. Der Rückstand wird mit Wasser bei 80°C digeriert, danach wird abfiltriert und getrocknet. Hierbei erhält man 81 g des Biscarbamates der oben angegebenen Formel (Fp. 186°C, nach $^1$HNMR- und $^{13}$CNMR-Spektren rein, Analyse ber. für $C_{12}H_{16}N_2O_4$ (%): C:57.1, H:6.4, O:25.4, N:11.1; gef.: C:57.0, H:6.4, O:25.3, N:11.0). Dies entspricht einer Ausbeute von 77.1 %.

**Patentansprüche**

1. Verfahren zur Herstellung von Biscarbamaten der allgemeinen Formel

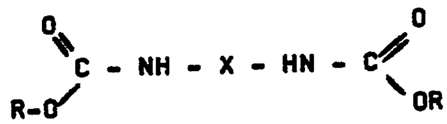 I,

in der R für einen Alkylrest mit 1 bis 8 C-Atomen steht und X einen Kohlenwasserstoffrest mit 2 bis 6O C-Atomen bedeutet, in dem ein oder mehrere C-Atome durch Heteroatome ersetzt sein können und der Halogenatome, Ester-, Carbonyl- oder Nitrilgruppen enthalten kann, dadurch gekennzeichnet, daß man Formamide der allgemeinen Formel

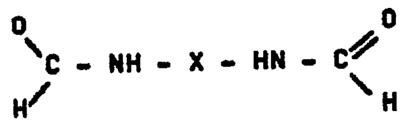 II,

in der X die obengenannte Bedeutung hat, in Gegenwart eines Alkanols der Formel ROH, in der R die obengenannte Bedeutung hat, und eines ionogenen Halogenids elektrochemisch oxidiert.

2. Verfahren nach Anspruch 1, dadurch gekennzeichnet, daß man als ionogenes Halogenid ein Salz der Bromwasserstoffsäure verwendet.

3. Verfahren nach Anspruch 1, dadurch gekennzeichnet, daß man als Anoden Graphitanoden verwendet.

4. Verfahren nach Anspruch 1, dadurch gekennzeichnet, daß man als Alkanol Methanol verwendet.

5. Verfahren nach Anspruch 1, dadurch gekennzeichnet, daß man für die elektrochemische Oxidation einen Elektrolyten einsetzt, dessen Gehalt an Formamid der Formel II 1 bis 5O Gew.%, an Alkanol der Formel ROH 4O bis 9O Gew.% und an ionogenem Halogenid O,1 bis 1O Gew.% beträgt.

**Claims**

1. A process for preparing a biscarbamate of the general formula

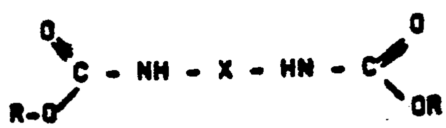 I,

where R is alkyl of 1 to 8 carbon atoms and X is a hydrocarbon radical of 2 to 60 carbon atoms in which one or more carbon atoms may be replaced by hetero atoms and which may contain halogen atoms or ester, carbonyl or nitrile groups, which comprises electrochemically oxidizing a formamide of the general formula

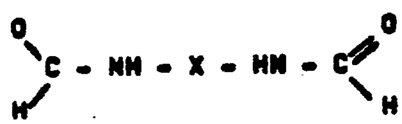 II,

where X has the abovementioned meaning, in the presence of an alkanol of the formula ROH, where R has the abovementioned meaning, and of an ionic halide.

2. A process as claimed in claim 1, wherein the ionic halide used is a salt of hydrobromic acid.

3. A process as claimed in claim 1, wherein the anode is made of graphite.

4. A process as claimed in claim 1, wherein the alkanol used is methanol.

5. A process as claimed in claim 1, wherein the electrochemical oxidation is carried out on an electrolyte which contains 1–50% by weight of formamide of the formula II, 40–90% by weight of alkanol of the formula ROH and 0.1–10% by weight of ionic halide.

**Revendications**

1. Procédé de préparation de biscarbamates de formule générale

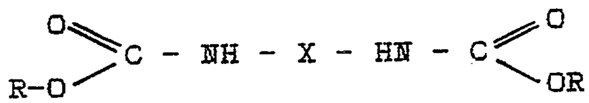 I

dans laquelle R est mis pour un reste alkyle à 1–8 atomes de carbone et X pour un reste hydrocarbure à 2–60 atomes de carbone dans lequel un ou plusieurs atomes de carbone peuvent être remplacés par des hétéroatomes et qui peut contenir des atomes d'halogène, des groupements ester, carbonyle ou nitrile, caractérisé en ce qu'on oxyde électrochimiquement des formamides de formule générale

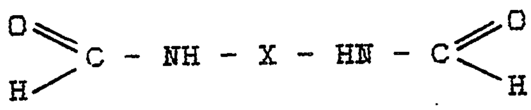 II

dans laquelle X a la signification donnée précédemment, en présence d'un alcanol de formule ROH, dans laquelle R a la signification donnée précédemment, et d'un halogénure ionogène.

2. Procédé selon la revendication 1, caractérisé en ce qu'on utilise, comme halogénure ionogène, un sel de l'acide bromhydrique.

3. Procédé selon la revendication 1, caractérisé en ce qu'on utilise, comme anodes, des anodes de graphite.

4. Procédé selon la revendication 1, caractérisé en ce qu'on utilise, comme alcanol, le méthanol.

5. Procédé selon la revendication 1, caractérisé en ce qu'on utilise, pour l'oxydation électrochimique, un électrolyte dont la teneur en formamide de formule II est comprise entre 1 et 50% en poids, la teneur en alcanol de formule ROH est comprise entre 40 et 90% en poids et la teneur en halogénure ionogène est comprise entre 0,1 et 10% en poids.